# EUROPEAN PATENT APPLICATION

(11) **EP 4 066 820 A1**
(43) Date of publication of application: **05.10.2022**
(21) Application number: 22164272.1
(22) Date of filing: 25.03.2022
(51) Int. Cl.: A61K 9/20, A61K 9/28

(54) **THE FILM COATED TABLET OF VILDAGLIPTIN AND METFORMIN HYDROCHLORIDE**

(30) Priority: 29.03.2021 TR 202105579
(71) Applicant: SANOVEL ILAC SANAYI VE TICARET A.S., Istanbul 34460 (TR)
(72) Inventor: TOMBAYOGLU, Vildan, Istanbul (TR); EMEN, Seyhmus, Istanbul (TR); PALANTOKEN, Arzu, Istanbul (TR); SUNEL, Fatih, Istanbul (TR)
(74) Representative: Sevinç, Erkan

(57) **Abstract**

The present invention relates to a film coated tablet comprising vildagliptin or vildagliptin HCl, at least one binder and a mixture comprising metformin hydrochloride, so the tablet provides the desired stability and pharmacotechnical properties and the desired dissolution profile. The present invention also relates to a simple, rapid, cost effective, time-saving and industrially convenient method of preparing the film coated tablet.

## Description

### Field of the Invention

The present invention relates to a film coated tablet comprising vildagliptin or vildagliptin HCI, at least one binder and a mixture comprising metformin hydrochloride, so the tablet provides the desired stability and pharmacotechnical properties and the desired dissolution profile. The present invention also relates to a simple, rapid, cost effective, time-saving and industrially convenient method of preparing the film coated tablet.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:
Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose.
In Type 2 diabetes, the pancreas makes insulin, but it either doesn't produce enough, or the insulin does not work properly. This diabetes occurs most often in people who are over 40 years old and overweight. Type 2 diabetes may sometimes be controlled with a combination of diet, weight management, and exercise. However, treatment also may include oral glucose-lowering medications or insulin injections.

Vildagliptin is a dipeptidyl dipeptidase-IV (DPP-IV) inhibitor developed for use in the treatment of type 2 diabetes (non-insulin dependent diabetes). Vildagliptin inhibits the degradation of the dipeptidyl dipeptidase-IV enzyme, thereby inhibiting the effects of incretin hormones, glucagon-like peptide-1 (GLP-1), and of glucose-dependent insulinotropic peptide (GIP). The chemical designation of vildagliptin is (S)-{[(3-hydroxyadamantan-1-yl)amino]acetyl}pyrrolidine-2-carbonitrile, with the chemical structure illustrated below in Formula 1.

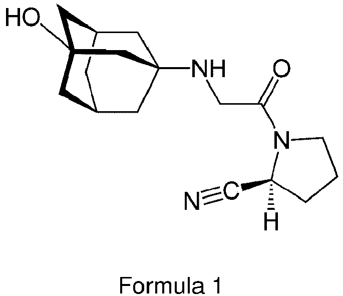

Vildagliptin is soluble in water and in organic polar solvents.

Vildagliptin is marketed under the trademark Galvus^{®} in 50 mg dosage forms. It is used against diabetes mellitus, but particularly in treating type 2 diabetes.

Metformin is antidiabetics having an orally-administrated biguanide structure. Metformin hydrochloride is a white to off-white crystalline compound and it is freely soluble in water and practically insoluble in acetone, ether and chloroform. Oral doses of metformin are generally recommended in the range of 500 to 2500 mg a day and a single dose may vary from 500 to 850 mg. It is used singly or in combination with sulfonylureas, alpha-glucosidase inhibitors, or insulin.

The chemical name of metformin hydrochloride is 1,1-dimethylbiguanide hydrochloride, has the following chemical structure of Formula II.

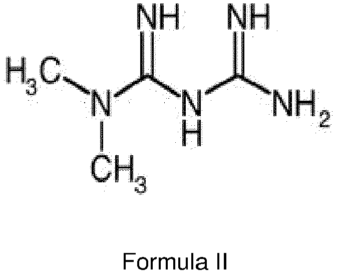

Combination product of vildagliptin and metformin hydrochloride is marketed under the trademark Eucreas^{®} (50mg/850mg and 50mg/1000mg dosage forms of vildagliptin and metformin hydrochloride). Pharmaceutical formulations of combined respective active ingredients and the process for their preparation are disclosed in EP 1948149.

Combination preparations of metformin and vildagliptin are described in WO 2007/041053. A tablet disclosed can contain, in addition to the active substances, usual excipients, for example fillers, binders, disintegrants, lubricants and colorants. Examples of lubricants that are mentioned are colloidal silica, magnesium trisilicate, starch, talc, calcium phosphate, magnesium stearate, aluminium stearate, calcium stearate, magnesium carbonate, magnesium oxide, polyethylene glycol, cellulose and microcrystalline cellulose.

EP2477660 (A1) discloses a pharmaceutical composition comprising an active agent metformin or its salt in combination with an active agent sitagliptin or vildagliptin or their salt, and a lubricant (more than 10 wt.%, based on the total weight of the composition). The lubricant is polyethylene glycol or a mixture of polyethylene glycol with at least one of the other lubricant.

Both metformin HCI and vildagliptin have some structural problems. Stability-related problems do occur in many active agents, including vildagliptin, under the influence of ambient and physical conditions. Vildagliptin is an active agent that is highly-susceptible to air and humidity. When vildagliptin is exposed to air and humidity, it degrades structurally and develops chemical behavioral changes. The stability of vildagliptin products developed is not at a desired level and the shelf life thereof is shortened. In addition, vildagliptin is reactive against the excipients employed in developing formulations containing the same. This fact causes impurities to occur in the formulation and leads to the inclusion of undesired components into the formulation. Also, the compressibility of metformin is poor. Considering these, it is very difficult to create a formulation comprising vildagliptin and metformin HCI.

In the present invention, a film coated tablet comprising vildagliptin or vildagliptin HCI, a mixture comprising metformin hydrochloride (the amount of metformin HCI in the mixture is between %92.0 and %97.0 by weight) and at least one binder, wherein the mixture further comprising at least one pharmaceutically acceptable excipient which is selected from the group comprising binders, fillers, lubricants or mixtures thereof. The film coated tablet with a certain proportion and selected excipients and a process of the tablet was created to overcome the above problems.

### Detailed Description of the Invention

The main object of the present invention is to provides a tablet comprising vildagliptin/vildagliptin HCI and metformin HCI capable of being compressed into a tablet having high stability.

Another object of the present invention is to provide a tablet, considering the negative structural features of metformin HCI and vildagliptin having the desired dissolution profile and compressibility.

According to one embodiment of the present invention, a film coated tablet comprises
- Vildagliptin or vildagliptin HCI
- A mixture comprising metformin hydrochloride,
- At least one binder,
wherein the amount of metformin HCI in the mixture is between %92.0 and %97.0 by weight and the mixture comprising at least one pharmaceutically acceptable excipient which is selected from the group comprising binders, fillers, lubricants or mixtures thereof.

According to one embodiment of the present invention, the amount of vildagliptin or vildagliptin HCI is between 2.0% and 10.0% by weight in the total formulation. Preferably, it is between 2.0% and 7.0% or 3.0% and 5.0% by weight in the total formulation.

According to one embodiment of the present invention, the amount of the mixture comprising metformin hydrochloride is between 70.0% and 85.0% by weight in the total formulation.

Metformin HCI is used big proportion that can lead to considerable problems during the preparation of formulation with regard to the uniformity of the content of active agent in the individual composition units. Because of problems uniformity of the content, the active substance may interact with several excipients. It reflects that content uniformity play important role in the dissolution of the drug. Also, in terms of physical properties, the compressibility of metformin is poor, and how to obtain a tablet or a capsule having an acceptable mechanical strength is an important problem in formulation development.

In the present invention, the amount of metformin HCI in the mixture is between %95.0 by weight (metformin granulate Direct Compressible grade %95). So, it helps to provide the desired uniformity of the content and therefore it provides the desired dissolution profile. Also, it ensures the desired compressibility and high physically. Thanks to the mixture, compressing of tablet can made by direct compression without extra process even if metformin had low compressibility.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation (especially the mixture) is developed. The formulations should have no physicochemical incompatibility between the active agents and the excipients.

Suitable filler in the mixture are selected from group comprising microcrystalline cellulose, lactose anhydrous, dicalcium phosphate dihydrate, ammonium alginate, calcium carbonate, calcium phosphate, calcium sulfate, cellulose, cellulose acetate, dextrates, dextrin, dextrose, erythritol, ethylcellulose, mannitol, magnesium carbonate, magnesium oxide, maltodextrin, polydextrose, polymethacrylates, sodium alginate, sodium chloride, starch, sugar spheres, sulfobutylether beta-cyclodextrin, polysorbate 80, xylitol or mixtures thereof.

According to one embodiment of the present invention, filler in the mixture is microcrystalline cellulose.

Suitable binder in the mixture are selected from the group comprising sodium carboxymethyl cellulose, polyvinylpyrrolidone (K30, K90), sodium carboxymethyl cellulose, polyethylene glycol, starch, pregelatinized starch, sodium alginate, hydroxypropyl methyl cellulose, hydroxypropyl cellulose, carboxy methyl cellulose, methyl cellulose, gelatin, carrageenan, guar gum, polymethacrylates, methacrylate polymers, hyaluronic acid, pectin, polysaccharides, carbomer, poloxamer, polyacrylamide, aluminium hydroxide, polyoxyethylene-alkyl ether, polydextrose, polyethylene oxide or mixtures thereof.

According to one embodiment of the present invention, binder in the mixture is sodium carboxymethyl cellulose or polyvinylpyrrolidone (K30, K90) or mixtures thereof.

Suitable lubricants in the mixture are selected from the group comprising magnesium stearate, sodium stearyl fumarate, calcium stearate, sodium chlorate, magnesium lauryl sulfate, sodium acetate, sodium benzoate, polyethylene glycol, sodium lauryl sulphate or mixtures thereof.

According to one embodiment of the present invention, lubricant in the mixture is magnesium stearate.

According to one embodiment of the present invention, the mixture comprises metformin HCI, microcrystalline cellulose, sodium carboxymethyl cellulose, polyvinylpyrrolidone K30, polyvinylpyrrolidone K90, magnesium stearate. This ensures the desired compressibility and high physically. Thanks to the mixture, compressing of tablet can made by direct compression without extra process even if metformin had low compressibility.

According to one embodiment of the present invention, the amount of metformin hydrochloride in the mixture is between 65.0% and 82.0% by weight in the total formulation, the amount of microcrystalline cellulose in the mixture is between 0.1% and 2.0% by weight in the total formulation, the amount of sodium carboxymethyl cellulose in the mixture is between 0.05% and 1.0% by weight in the total formulation, the amount of polyvinylpyrrolidone K30 in the mixture is between 1.5% and 3.5% by weight in the total formulation, the amount of polyvinylpyrrolidone K90 in the mixture is between 0.1% and 1.0% by weight in the total formulation, the amount of magnesium stearate in the mixture is between 0.1% and 1.0% by weight in the total formulation.

Suitable binders are selected from the group comprising copovidone, polyvinylpyrrolidone, sodium carboxymethyl cellulose, polyethylene glycol, polyvinyl alcohol, starch, pregelatinized starch, glucose, glucose syrup, natural gums, sucrose, sodium alginate, hydroxypropyl methyl cellulose, hydroxypropyl cellulose, carboxy methyl cellulose, methyl cellulose, gelatin, carrageenan, guar gum, carbomer, polymethacrylates, methacrylate polymers, gelatin, agar, alginate, alginic acid, xanthan gum, hyaluronic acid, pectin, polysaccharides, carbomer, poloxamer, polyacrylamide, aluminium hydroxide, laponit, bentonit, polyoxyethylene-alkyl ether, polydextrose, polyethylene oxide or mixtures thereof.

According to one embodiment of the present invention, the amount of binders is between 4.0% and 25.0% by weight in the total formulation.

According to one embodiment of the present invention, the binder is copovidone or polyvinylpyrrolidone or mixtures thereof.

According to one embodiment of the present invention, surprisingly, using copovidone provides form hydrogen bonds with vildagliptin, the moisture sensitive drug, increasing thereby their solubility, dissolution rate, and stability.

According to one embodiment of the present invention, especially, copovidone S- Ultra is used as binder. For its peroxide ratio is lower than other copovidone, copovidone S- Ultra helps to provide the desired stability.

According to one embodiment of the present invention, the tablet comprises copovidone as binder and the amount of copovidone is between 15.0% and 25.0% by weight in the total formulation

According to one embodiment of the present invention, the tablet comprises copovidone and polyvinylpyrrolidone as binder and the amount of copovidone is between 2.0% and 6.0% by weight in the total formulation, the amount of polyvinylpyrrolidone is between 2.0% and 6.0% by weight in the total formulation.

According to one embodiment of the present invention, the film coated tablet comprises
- Vildagliptin or vildagliptin HCI
- A mixture comprising metformin hydrochloride,
- copovidone and/or polyvinylpyrrolidone,
wherein the amount of metformin HCI in the mixture is between %92.0 and %97.0 by weight and the mixture comprising metformin HCI, microcrystalline cellulose, sodium carboxymethyl cellulose, polyvinylpyrrolidone K30, polyvinylpyrrolidone K90, magnesium stearate.

As used here in, 'particle size' means the cumulative volume size distrubition as tested by any conventionally accepted method such as the laser diffraction method (i.e. malvern analysis). The term d (0.9) means, the size at which 90% by volume of the particles are finer.

According to this embodiment of the present invention, vildagliptin or vildagliptin HCI has a d (0.9) particle size less than 600 µm, preferably between 400 µm and 600 µm, more preferably between 500 µm and 600 µm

According to this embodiment of the present invention, metformin HCI has a d (0.9) particle size less than 500 µm, preferably between 200 µm and 500µm, more preferably between 300 µm and 500 µm.

The film coated tablet of the present invention may be prepared, using standard techniques and manufacturing processes well known in the art, such as direct compression or dry granulation.

According to one embodiment of the present invention, the film coated tablet is obtained by using direct compression, it is a simple and low-cost production method was employed. Also, since vildagliptin is highly-susceptible to air and humidity, this method is advantage because it does not contain high humidity environment and it is fast.

According to one embodiment of the present invention, a process for the preparation of the film coated tablet further comprises the following steps:
a) Sieving the mixture comprising metformin HCI through a 1.0 mm sieve,
b) Sieving vildagliptin and copovidone and /or polyvinylpyrrolidone through a 0.9 mm sieve and adding half of the mixture comprising metformin HCI at step (a) and then mixing,
c) Adding the remaining of the mixture comprising metformin HCI and then mixing,
d) Compressing to form of a tablet,
e) Coating tablets with coating.

According to one embodiment of the present invention, the final product has 1.0% moisture limit.

According to one embodiment of the present invention, during the tablet compressing process, dry air supply and air outlet system has been established to reduce the moisture of tablets. Also, after the tablet compressing, during the coating dry air supply system has been established and the humidity of the supplied air is reduced to less than 10%.

Desiccant was used to prevent moisture absorption until the tablet compressing process was completed. Desiccants are incorporated in IBC. Silica gel as desiccant was placed in the areas where the product was kept and the product was transported. It were not come into contact with the product.

### Example 1: The tablet formulation comprising vildagliptin and metformin HCl

| **Ingredients** | | **% by weight** |
|---|---|---|
| Vildagliptin | | 2.0 - 10.0 |
| **A mixture** | | 70.0 - 85.0 |
| | • Metformin HCI | |
| | • Microcrystalline cellulose | |
| | • Sodium carboxymethyl cellulose | |
| | • Polyvinylpyrrolidone K30 | |
| | • Polyvinylpyrrolidone K90 | |
| | • Magnesium stearate | |
| Copovidone | | 2.0 - 6.0 |
| Polyvinylpyrrolidone | | 2.0 - 6.0 |
| Coating | | 1.0 - 5.0 |
| **TOTAL** | | **100** |

### Example 2: The tablet formulation comprising vildagliptin and metformin HCl

| **Ingredients** | **% by weight** |
|---|---|
| Vildagliptin | 3.98 |
| A mixture comprising metformin HCI | 83.87 |
| Copovidone | 4.78 |
| Polyvinylpyrrolidone | 4.58 |
| Coating | 2.79 |
| **TOTAL** | **100** |

### Example 3: The tablet formulation comprising vildagliptin and metformin HCI

| **Ingredients** | **% by weight** |
|---|---|
| Vildagliptin | 4.59 |
| A mixture comprising metformin HCI | 82.09 |
| Copovidone | 5.41 |
| Polyvinylpyrrolidone | 5.16 |
| Coating | 2.75 |
| **TOTAL** | **100** |

### A process for example 1 or 2 or 3;

a) Sieving the mixture through a 1.0 mm sieve,
b) Sieving vildagliptin, copovidone and polyvinylpyrrolidone through a 0.9 mm sieve and adding half of the mixture at step (a) and then mixing,
c) Adding the remaining of the mixture and then mixing,
d) Compressing to form of a tablet,
e) Coating tablets with coating (described example 8).

### Example 4: The tablet formulation comprising vildagliptin and metformin HCl

| **Ingredients** | | **% by weight** |
|---|---|---|
| Vildagliptin | | 2.0 - 10.0 |
| A mixture | | 70.0 - 85.0 |
| | • Metformin HCI | |
| | • Microcrystalline cellulose | |
| | • Sodium carboxymethyl cellulose | |
| | • Polyvinylpyrrolidone K30 | |
| | • Polyvinylpyrrolidone K90 | |
| | • Magnesium stearate | |
| Copovidone | | 15.0 - 25.0 |
| Coating | | 1.0 - 5.0 |
| **TOTAL** | | **100** |

### Example 5: The tablet formulation comprising vildagliptin and metformin HCI

| **Ingredients** | **% by weight** |
|---|---|
| Vildagliptin | 3.61 |
| A mixture comprising metformin HCI | 76.0 |
| Copovidone | 17.86 |
| Coating | 2.53 |
| **TOTAL** | **100** |

### Example 6: The tablet formulation comprising vildagliptin and metformin HCl

| **Ingredients** | **% by weight** |
|---|---|
| Vildagliptin | 3.98 |
| A mixture comprising metformin HCI | 71.29 |
| Copovidone | 22.33 |
| Coating | 2.39 |
| **TOTAL** | **100** |

### A process for example 4 or 5 or 6;

a) Sieving the mixture through a 1.0 mm sieve,
b) Sieving vildagliptin and copovidone through a 0.9 mm sieve and adding half of the mixture at step (a) and then mixing,
c) Adding the remaining of the mixture and then mixing,
d) Compressing to form of a tablet,
e) Coating tablets with coating (described example 8).

### Example 7: A mixture comprising metformin HCl

| **Ingredients** | **% by weight** |
|---|---|
| Metformin HCI | 92.0 - 97.0 |
| Microcrystalline cellulose | 0.1 -2.0 |
| Sodium carboxymethyl cellulose | 0.01 - 1.0 |
| Polyvinylpyrrolidone K30 | 1.5-3.10 |
| Polyvinylpyrrolidone K90 | 0.01 - 1.0 |
| Magnesium stearate | 0.01 - 1.0 |
| **TOTAL** | **100** |

### Example 8: A mixture comprising metformin HCl

| **Ingredients** | **% by weight** |
|---|---|
| Metformin HCI | 95.0 |
| Microcrystalline cellulose | 1.0 |
| Sodium carboxymethyl cellulose | 0.2 |
| Polyvinylpyrrolidone K30 | 2.85 |
| Polyvinylpyrrolidone K90 | 0.475 |
| Magnesium stearate | 0.475 |
| **TOTAL** | **100** |

### Example 9: Coating

| **Ingredients** | **% by weight in a coating** | **% by weight in a coating** |
|---|---|---|
| Copovidone S-630 | 30.47 | 30.47 |
| Hydroxypropyl cellulose | 8.70 | 8.70 |
| Polydextrose | 13.04 | 13.04 |
| Talc | 9.74 | 9.74 |
| Polyethylene glycol | 8.70 | 8.70 |
| Medium chain triglycerides | 4.35 | 4.35 |
| Titanium dioxide | 15.84 | 19.89 |
| Yellow iron oxide | 9.15 | 5.10 |
| Red iron oxide | 0.005 | 0.005 |
| Black iron oxide | 0.005 | 0.005 |
| **TOTAL** | **100** | **100** |

## Claims

1. A film coated tablet comprising;
- Vildagliptin or vildagliptin HCI
- A mixture comprising metformin hydrochloride,
- At least one binder,
wherein the amount of metformin HCI in the mixture is between %92.0 and %97.0 by weight and the mixture comprising at least one pharmaceutically acceptable excipient which is selected from the group comprising binders, fillers, lubricants or mixtures thereof.

2. The film coated tablet according to claim 1; wherein the amount of vildagliptin or vildagliptin HCI is between 2.0% and 10.0% by weight in the total formulation.

3. The film coated tablet according to claim 1; wherein the amount of the mixture comprising metformin hydrochloride is between 70.0% and 85.0% by weight in the total formulation.

4. The film coated tablet according to claim 1; wherein the amount of metformin HCI in the mixture is between %95.0 by weight.

5. The film coated tablet according to claim 1; wherein filler in the mixture are selected from group comprising microcrystalline cellulose, lactose anhydrous, dicalcium phosphate dihydrate, ammonium alginate, calcium carbonate, calcium phosphate, calcium sulfate, cellulose, cellulose acetate, dextrates, dextrin, dextrose, erythritol, ethylcellulose, mannitol, magnesium carbonate, magnesium oxide, maltodextrin, polydextrose, polymethacrylates, sodium alginate, sodium chloride, starch, sugar spheres, sulfobutylether beta-cyclodextrin, polysorbate 80, xylitol or mixtures thereof.

6. The film coated tablet according to claim 5; wherein filler in the mixture is microcrystalline cellulose.

7. The film coated tablet according to claim 1; wherein binder in the mixture are selected from the group comprising sodium carboxymethyl cellulose, polyvinylpyrrolidone (K30, K90), sodium carboxymethyl cellulose, polyethylene glycol, starch, pregelatinized starch, sodium alginate, hydroxypropyl methyl cellulose, hydroxypropyl cellulose, carboxy methyl cellulose, methyl cellulose, gelatin, carrageenan, guar gum, polymethacrylates, methacrylate polymers, hyaluronic acid, pectin, polysaccharides, carbomer, poloxamer, polyacrylamide, aluminium hydroxide, polyoxyethylene-alkyl ether, polydextrose, polyethylene oxide or mixtures thereof.

8. The film coated tablet according to claim 7; wherein binder in the mixture is sodium carboxymethyl cellulose or polyvinylpyrrolidone or mixtures thereof.

9. The film coated tablet according to claim 1; wherein lubricants in the mixture are selected from the group comprising magnesium stearate, sodium stearyl fumarate, calcium stearate, sodium chlorate, magnesium lauryl sulfate, sodium acetate, sodium benzoate, polyethylene glycol, sodium lauryl sulphate or mixtures thereof.

10. The film coated tablet according to claim 1; wherein the mixture comprising metformin HCI, microcrystalline cellulose, sodium carboxymethyl cellulose, polyvinylpyrrolidone K30, polyvinylpyrrolidone K90, magnesium stearate.

11. The film coated tablet according to claim 1; wherein binders are selected from the group comprising copovidone, polyvinylpyrrolidone, sodium carboxymethyl cellulose, polyethylene glycol, polyvinyl alcohol, starch, pregelatinized starch, glucose, glucose syrup, natural gums, sucrose, sodium alginate, hydroxypropyl methyl cellulose, hydroxypropyl cellulose, carboxy methyl cellulose, methyl cellulose, gelatin, carrageenan, guar gum, carbomer, polymethacrylates, methacrylate polymers, gelatin, agar, alginate, alginic acid, xanthan gum, hyaluronic acid, pectin, polysaccharides, carbomer, poloxamer, polyacrylamide, aluminium hydroxide, laponit, bentonit, polyoxyethylene-alkyl ether, polydextrose, polyethylene oxide or mixtures thereof.

12. The film coated tablet according to claim 11; wherein the binder is copovidone or polyvinylpyrrolidone or mixtures thereof.

13. The film coated tablet according to claim 1; wherein the film coated tablet comprises
- Vildagliptin or vildagliptin HCI
- A mixture comprising metformin hydrochloride,
- copovidone and/or polyvinylpyrrolidone,
wherein the amount of metformin HCI in the mixture is between %92.0 and %97.0 b y weight and the mixture comprising metformin HCI, microcrystalline cellulose, sodium carboxymethyl cellulose, polyvinylpyrrolidone K30, polyvinylpyrrolidone K90, magnesium stearate.

14. A process for the preparation of the film coated tablet comprising the following steps:
a) Sieving the mixture comprising metformin HCI through a 1.0 mm sieve,
b) Sieving vildagliptin and copovidone and /or polyvinylpyrrolidone through a 0.9 mm sieve and adding half of the mixture comprising metformin HCI at step (a) and then mixing,
c) Adding the remaining of the mixture comprising metformin HCI and then mixing,
d) Compressing to form of a tablet,
e) Coating tablets with coating.
